# EUROPEAN PATENT SPECIFICATION

(11) **EP 0 577 225 B1**
(45) Date of publication and mention of the grant of the patent: **01.12.2004**
(21) Application number: 93202324.5
(22) Date of filing: 16.01.1987
(51) Int. Cl.: A61K 49/16, A61K 39/395

(54) **Diagnosis and treatment of inflammation**
Diagnose und Behandlung von Entzündung
Diagnostic et traitement d'inflammation

(30) Priority: 16.01.1986 US 819585
(43) Date of publication of application: 05.01.1994
(62) Divisional of application: 87300395.8
(73) Proprietor: THE GENERAL HOSPITAL CORPORATION, Boston, MA 02114 (US)
(72) Inventor: Rubin, Robert H., Brookline, Massachusetts 02146 (US); Strauss, William H., Skillman, New Jersey 08558 (US)
(74) Representative: Bassil, Nicholas Charles

(56) References cited:
- EP-A- 0 137 457
- EP-A- 0 154 550
- EP-A- 0 178 125
- WO-A-86/02363
- WO-A-86/03754
- WO-A-87/04351
- WO-A-87/06616
- WO-A-88/02028
- GB-A- 2 004 464
- GB-A- 2 101 407
- US-A- 4 315 906
- US-A- 4 472 371
- US-A- 4 522 918
- US-A- 4 634 586

## Description

This invention is directed to substances for use in diagnosing inflammation sites in an individual.

Inflammation occurs as a consequence of tissue damage. This tissue damage can result from microbial invasion, auto-immune processes, tissue infection, allograft rejection, or such hurtful or destructive external influences as heat, cold, radiant energy, electrical or chemical stimuli, or mechanical trauma. Whatever the cause or bodily site, the inflammatory response is quite similar, consisting of a complicated set of functional and cellular adjustments, involving the microcirculation, fluid shifts, and inflammatory cells (leukocytes). When tissue damage occurs, soluble chemical substances are elaborated which initiate the inflammatory response. The inflammatory response consists of a complex series of events which may be summarized as:
1. A local increase in blood-flow, with capillary dilatation and increased permeability to the fluid components of the blood;
2. A localized exudation of fluid at the site of injury, including the proteins of the plasma that normally leave the capillaries at a relatively low rate;
3. The exudation of leukocytes from the capillaries into the inflammation site. This exudate initially consists primarily of polymorphonuclear leukocytes, followed by monocytes, lymphocytes, and plasma cells. These leukocytes produce a variety of mediators that control the extent and duration of the inflammatory response, and have a series of receptors on their surfaces available to react to the host of chemical mediators and proteins that are part of the inflammatory fluid. Such leukocyte receptor-mediator or protein interactions are important in controlling leukocyte function within the inflammatory site.

The identification and characterization of the sites of inflammation are an important part of medical and veterinary practice. In the case of infectious causes of inflammation, it is frequently necessary to search for "hidden sites of inflammation" in individuals who present with clinical syndromes no more specific than fever and weight loss. Similarly, in patients with auto-immune disease such as rheumatoid arthritis or allograft rejection as causes of inflammation, identification of the site(s) and extent of inflammation and its changes with therapy are an important part of medical and veterinary practice. Not surprisingly, then, much effort has been expended and many techniques developed in an attempt to assess the site(s) and extent of the inflammatory process. These techniques include conventional x-ray techniques, computerized axial tomographic scanning (CAT scanning), and a variety of radionuclide scans. (Sutt-on, A Textbook of Radiology and Imaging, 3rd Ed., Churchill Livingston, 1980; Clinical Nuclear Medicine, Maysey et al., ed., W.B. Sanders, 1983.) Examples of radionuclide scans which have been utilized are:
1. ⁶⁷Gallium, which when injected into an animal or a human binds to the plasma protein transferrin and tends to localize at sites of chronic inflammation.
2. ¹¹¹Indium labeled endogenous granulocytes, which when re-injected into the host will tend to accumulate at the site of inflammation; and
3. Radiolabeled chelates which pass into the extracellular fluid and can possibly then accumulate at such sites of fluid accumulation as those associated with inflammation;
4. Thallium scan or so-called first pass radionuclide angiogram to assess areas of increased blood flow.

All of these techniques, on occasion, may provide useful information, but are not adequate because of both false positive and false negative results. A more sensitive and specific means of delineating the anatomical localization of sites of inflammation, particularly one that could be performed serially to assess the response to therapy, is greatly to be desired.

GB 2004464 describes the use of radioactively labelled serum albumin in the quantitative measurement of the anti-inflammatory action of drugs. EP 0178125 describes bi-functional coupling agents for joining radionuclide metal ions to biologically useful molecules, including antibody Fab fragments. US 4315906 describes a preparation comprising affinity purified biologically active cold insoluble globulin having a specific opsonic activity of at least 1,000 units per mg of cold insoluble globulin protein. US 4522918 describes a process for producing antibodies from hybridoma cultures demonstrating a reactivity with human breast cancer. US 4634586 describes a leukocyte radioimmunoimaging reagent comprising an immunoreactive nonleukocidal conjugate of an anti-leukocyte antibody and a gamma emitting radioactive metal chelate. WO 87/04351 describes a method of detecting an inflammation site in an individual by administering to the individual a diagnostically effective amount of detectably labelled immunoglobulin or fragment thereof, wherein the immunoglobulin substantially accumulates at the site when the site is inflamed. EP 0137457 describes conjugates of target seeking biologically active molecules and metallothionein in which all or part of the metal in the metallothionein is suitable for diagnostic or therapeutic applications. US 4472371 describes labelled antibodies to anti-human tumour associated antigens, e.g. human carcinoembryonic antigen (CEA). EP 0154550 describes monoclonal antibodies capable of binding to pancreatic carcinomas of ductal origin, but not with pancreatic carcinoma of endocrine origin or regenerative, or normal pancreatic cells or pancreatitis cells. WO 88/02028 describes a monoclonal antibody to a penicillin binding protein. WO 86/03754 describes anti-Pseudomonas aeruginosa human monoclonal antibodies which recognise the lipopolysaccharide of P. aeruginosa. WO 87/06616 describes monoclonal antibodies to the genus *Bacteroides.* WO 86/02363 describes monoclonal antibodies to the genus *Bacillus.* GB 2109407 describes a monoclonal antibody to a tumour associated antigen to which a metallic radionuclide is bound directly or through a chelating agent, conjugated with the antibody.

The present invention relates to substances for use in a substantially non-invasive method of diagnosing sites of inflammation.

The present inventors have discovered that when immunoglobulins are allowed to contact both inflamed and non-inflamed sites in an individual, they tend to accumulate at the inflamed sites. Further, it was surprisingly discovered that the accumulation at the site of inflammation is not dependent upon the epitopic specificity of the immunoglobulins used. This effect, the concentration of immunoglobulin at the site of inflammation and its use in diagnostic imaging, has not been previously recognized. In other words, non-specific immunoglobulins or mixtures thereof can be used.

The present invention thus relates to the preparation of substances for use in detecting an inflammation site in an individual, the substance comprising a detectably labeled immunoglobulin or fragment thereof, wherein the immunoglobulin substantially accumulates at the site when the site is inflamed.

According to a first aspect of the invention, there is provided the use of one or more monoclonal antibodies in the preparation of a detectably labelled imaging agent for the diagnosis of inflammation, wherein the antibody or antibodies substantially accumulate at sites of inflammation, but wherein there is no substantial Fab binding to the antigen epitopes at said sites of inflammation.

According to a second aspect of the invention there is provided the use of one or more Fc fragments of monoclonal antibodies which accumulate at sites of inflammation in the preparation of a detectably labelled imaging agent for the diagnosis of inflammation, wherein said Fc fragments of monoclonal antibodies have substantially no epitopic specificity for said inflamed sites.

Preferably the immunoglobulin does not substantially accumulate at said site when said site is not inflamed.

The detectable label may be a radioactive isotope (such as ^{99m}Tc, ¹²³I, ¹³¹I, ¹¹¹In, ⁹⁷Ru, ⁶⁷Cu, ⁶⁷Ga, ⁶⁸Ga, ⁷²As, ⁸⁹Zr, and ²⁰¹Tl) or a paramagnetic label (such as ¹⁵⁷Gd, ⁵⁵Mn, ¹⁶²Dy, ⁵²Cr, and ⁵⁶Fe). When the label is paramagnetic, it may be detected by magnetic resonance imaging or positron emission tomography.

Administration of the labelled immunoglobulin may be parenteral, for example by intradermal, subcutaneous, intramuscular, intraperitoneal, or intravenous injection. Alternatively or in addition it may be by gradual perfusion, for example by intravenous or peristaltic means.

The imaging agents prepared in accordance with the first or second aspect of the invention are used in detecting the underlying cause of an inflammatory response in vivo in an individual, which comprises:
a) administering to said individual a diagnostically effective amount of the first labelled immunoglobulin or fragment;
b) imaging said individual to detect the presence of a site of inflammation;
c) based on the presence of a site of inflammation as demonstrated in step b, further administering to said individual the second labelled antibody or fragment; and
d) imaging said individual to detect the presence of the detectably labelled specific antibody or fragment thereof bound to the underlying cause of the inflammation at the site of inflammation.

The underlying cause of the inflammatory response may be treated therapeutically with an appropriate agent. The treatment may utilize a therapeutically labelled antibody specific for said underlying cause of the inflammatory response, bound to said agent. The specific antibody may be monoclonally derived. The agent may be a drug, a lectin (such as the alpha-chain of ricin), a toxin (such as diphtheria toxin) or a radioactive isotope (such as ¹²⁵I, ¹³¹I, ⁹⁰Y, ⁶⁷Cu, ²¹⁷Bi, ²¹¹At, ²¹²Pb, ⁴⁷Sc, or ¹⁰⁹Pd).

The individual may be a human. The inflammation may be a result of a microbial infection, a viral infection, a trauma, an autoimmune process, or a tumour.

The agent may be an antimicrobial such as an antibacterial, an anti-fungal, an anti-viral, or an antiparasitic.

### DESCRIPTION OF THE EXEMPLARY DRAWINGS

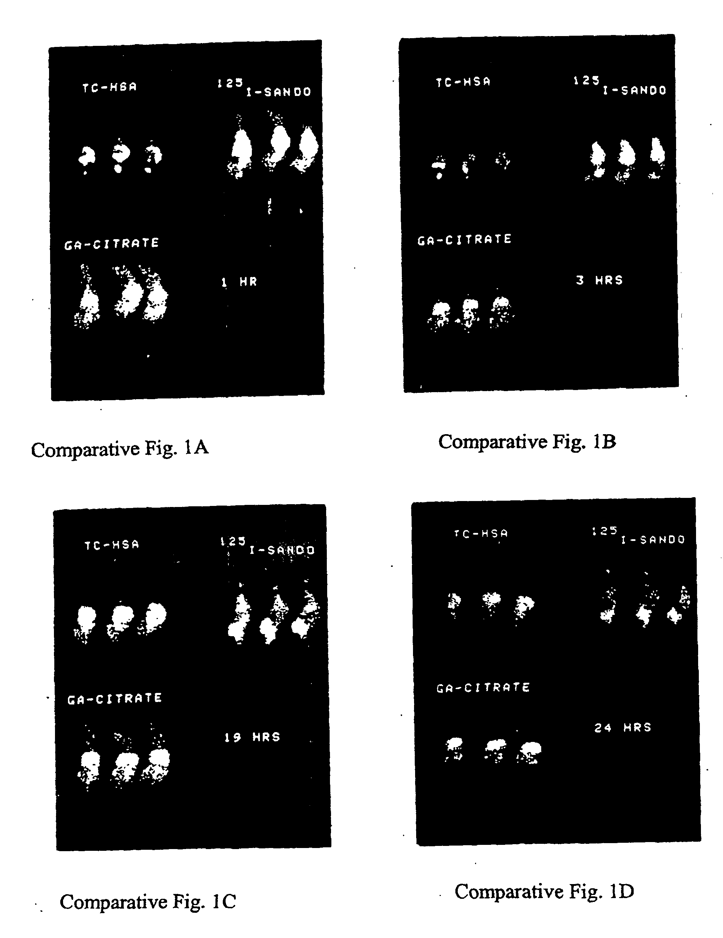

### Comparative Figure 1: Imaging of inflammation with radiolabeled preparations.

Comparative imaging results of three animals with inflammation due to injection of viable bacteria in the left thigh. Each animal was injected with a mixture of three radiolabeled preparations and imaged at various times.

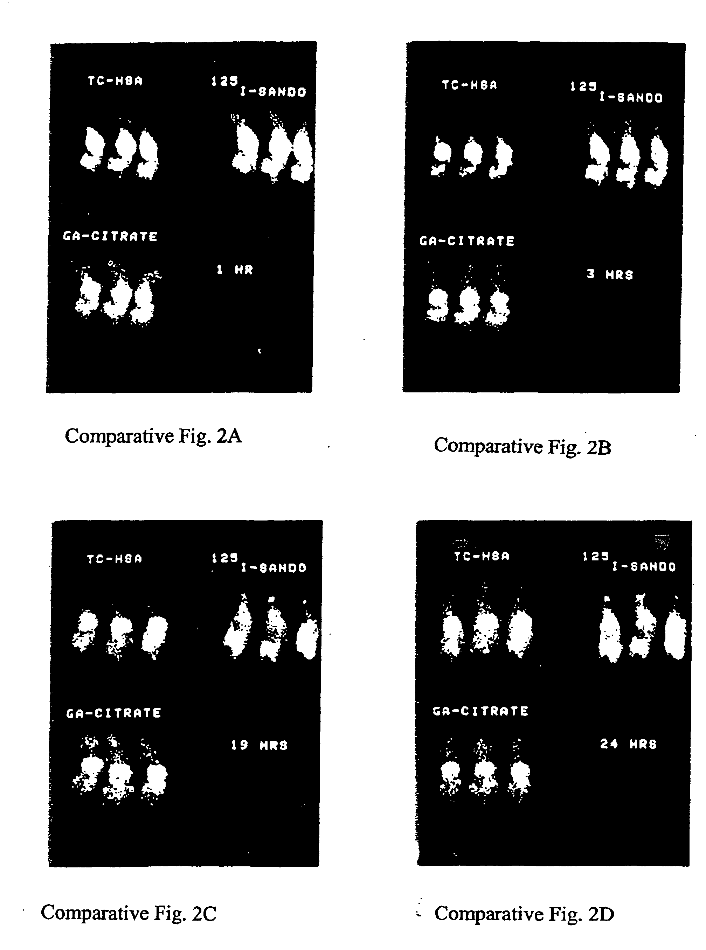

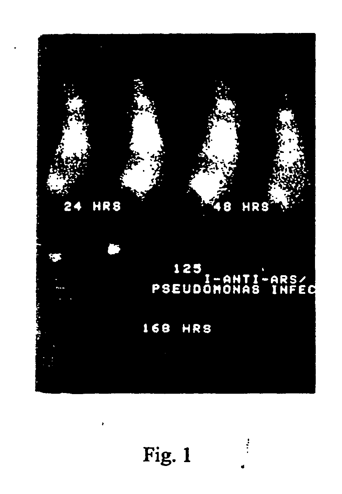

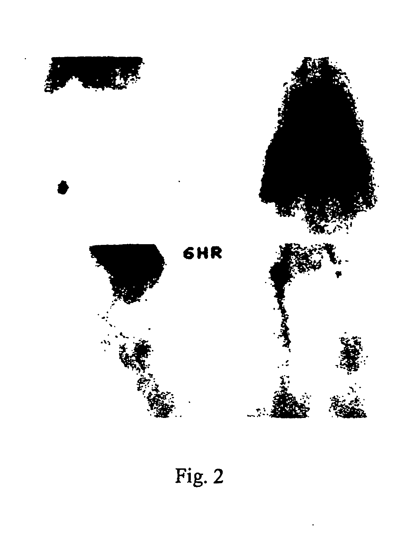

### Comparative Figure 2: Imaging of inflammation with radiolabeled preparations.

Comparative imaging results of three animals with inflammation due to injection of viable bacteria in the left thigh. Each animal was injected with a mixture of three radiolabeled preparations and imaged at various times.

### Figure 1: Imaging of inflammation with non-specific murine immunoglobulin.

This figure shows the kinetics of radiolabeled immunoglobulin in two animals with inflammatory sites in the left thigh due to injection of viable bacteria. Although the inflammation site is clearly detectable at 24 and 48 hours post-injection, the site is no longer detected by 160 hours post-injection.

### Figure 2: Imaging of inflammation in human patient.

Immunoglobulin scan of human patient showing localization of mycotic aneurysm in femoral graft.

### Figure 3: Imaging of inflammation in human patient.

Immunoglobulin scan of human patient indicating a diverticular abscess.

### Figure 4: Imaging of inflammation in human patient.

Immunoglobulin scans pre- and post-treatment of human patient with enteritis demonstrating the absence of inflammation after treatment.

### Figure 5: Imaging of inflammation in human patient.

Diagnosis of site of inflammation in groin of human patient by radiolabeled immunoglobulin imaging technique.

### DESCRIPTION OF THE PREFERRED EMBODIMENTS

The terms "inflammation" or "inflamed site" are used to denote conditions occurring in an individual due to tissue damage, regardless of the underlying cause or etiology. The concept is more fully described above in the Background Art.

The term "individual" is meant to include both animals and humans.

The immunoglobulins of the invention are monoclonally derived, the important factor being that the epitopic specificity of the immunoglobulin used is irrelevant to enable it to localize at the inflammation site.

Monoclonal immunoglobulins which can be used according to the method of the invention can be prepared using hybridoma fusion techniques (Kohler et al., European Journal of Immunology 6:292, 1976) or can be derived from known secreting myeloma cell lines such as those available from depositories such as the American Type Culture Collection. No antigenic or epitopic specificity is needed for the monoclonal immunoglobulin preparation to function effectively in this method. As a consequence, monoclonals of any specificity can be used.

In detecting an in vivo inflammation site in an individual, the detectably labeled immunoglobulin is advantageously given in a dose which is diagnostically effective. The term "diagnostically effective" means that the amount of detectably labeled immunoglobulin administered is sufficient to enable detection of the site of inflammation when compared to the background signal.

Generally, the dosage of detectably labeled immunoglobulin for diagnosis will vary depending on considerations such as age, condition, sex, and extent of disease in the patient, counterindications, if any, and other variables, to be adjusted by the individual physician. Dosage can vary from 0.01 mg/kg to 2,000 mg/kg, preferably 0.1 mg/kg to 1,000 mg/kg.

The term "immunoglobulin or a fragment thereof" as used in this invention is meant to include intact molecules as well as fragments thereof, such as, for example, the Fc fragment, which is capable of accumulating at the site of inflammation.

Similarly, the term "Fc portion or part thereof" as used in this invention is meant to include intact Fc fragments as well as portions of the Fc fragment capable of accumulating at the site of inflammation.

The term "diagnostically labeled" means that the immunoglobulin has attached to it a diagnostically detectable label.

There are many different labels and methods of labeling known to those of ordinary skill in the art. Examples of the types of labels which can be used in the present invention include radioactive isotopes and paramagnetic isotopes. Those of ordinary skill in the art will know of other suitable labels for binding to the immunoglobulins used in the invention, or will be able to ascertain such, using routine experimentation. Furthermore, the binding of these labels to the immunoglobulin can be done using standard techniques common to those of ordinary skill in the art.

For diagnostic in vivo imaging, the type of detection instrument available is a major factor in selecting a given radionuclide. The radionuclide chosen must have a type of decay which is detectable for a given type of instrument. In general, any conventional method for visualizing diagnostic imaging can be utilized in accordance with this invention.

Another important factor in selecting a radionuclide for in vivo diagnosis is that the half-life of a radionuclide be long enough so that it is still detectable at the time of maximum uptake by the target, but short enough so that deleterious radiation upon the host is minimized. Ideally, a radionuclide used for in vivo imaging will lack a particulate emission, but produce a large number of photons in a 140-200 keV range, which may be readily detected by conventional gamma cameras.

For in vivo diagnosis, radionuclides may be bound to immunoglobulin either directly or indirectly by using an intermediary functional group. Intermediary functional groups which are often used to bind radioisotopes which exist as metallic ions to immunoglobulins are diethylenetriaminepentaacetic acid (DTPA) and ethylenediaminetetracetic acid (EDTA). Typical examples of metallic ions which can be bound to immunoglobulins are ^{99m}Tc, ¹²³I, ¹¹¹In ¹³¹I, ⁹⁷Ru, ⁶⁷Cu, ⁶⁷Ga, ¹²⁵I, ⁶⁸Ga, ⁷²As, ⁸⁹Zr, and ²⁰¹Tl.

The immunoglobulins used in the method of the invention can also be labeled with paramagnetic isotypes for purposes of in vivo diagnosis. Elements which are particularly useful (as in Magnetic Resonance Imaging (MRI) techniques) in this manner include ¹⁵⁷Gd, ⁵⁵Mn, ¹⁶²Dy, ⁵²Cr, and ⁵⁶Fe.
Alternatively, the imaging agents prepared in accordance with the uses of the invention can be used to monitor the course of inflammation in an individual. sites it would be possible to determine whether a particular therapeutic regimen aimed at ameliorating the cause of the inflammatory process, or the inflammatory process itself, is effective.

Another embodiment of the invention includes diagnosing the specific underlying cause of the inflammation at the site. An individual suspected of having an inflammatory site is first administered a diagnostically effective amount of immunoglobulin, as previously described. This detectably labeled immunoglobulin may be of the same or different species as the individual to which it is being administered. The individual suspected of having an inflammatory site is then imaged to determine the presence of a site of inflammation. If the individual is found to have an inflammatory site, the individual is then given an antibody preparation(s) specific for the underlying cause of the inflammation which is suspected. This specific antibody can be from an individual of the same, or a different, species to that of the individual having the inflammatory site. After determining the specific cause of the inflammatory site it is then possible to administer a therapeutic agent, such as therapeutically labeled antibody specific for the underlying cause of the inflammatory process at the inflammation site.

There are distinct advantages to this embodiment of the invention, utilizing the sequential use of non-specific immunoglobulin from an individual of the same species as that suspected of having the inflammatory site, followed by the administration of specific antibody to define and identify the nature of the underlying cause of the inflammatory response. The potential advantages of this sequential strategy include the following:
1. The immunoglobulin preparation used to determine whether any inflammation at all is present is non-sensitizing to the recipient individual since it is from an individual of the same species. Thus, there is little in the way of an adverse allergic or immunologic reaction by the recipient to the immunoglobulin preparation.
2. If the inflammation is not found with the immunoglobulin preparation of 1. above, it is not necessary to administer the detectably labeled specific antibody. Thus, in the situation where detectably labeled specific antibody is unavailable from an individual of the same species as that of the recipient, the recipient will not be exposed to potentially sensitizing amounts of foreign antibody protein. At present, specific antibodies, particularly monoclonal antibodies, are chiefly of murine origin and, thus, may possibly excite an adverse immunologic response in a non-murine recipient. Such adverse immunologic responses limit the number of times a given recipient individual can be exposed to a specific antibody preparation from a different species. Therefore, exposure to such specific antibody of a different species should be restricted to situations of maximum clinical benefit.

The term "therapeutically conjugated" means that a specific antibody used as above described is conjugated to a therapeutic agent. The therapeutic agents used in this matter act directly upon the underlying cause of the inflammation. Examples of therapeutic agents which can be coupled to the specific antibodies used according to the method of the invention are drugs, radioisotopes, lectins, toxins, and antimicrobials.

Lectins are proteins, usually isolated from plant material, which bind to specific sugar moieties. Many lectins are also able to agglutinate cells and stimulate lymphocytes. Ricin is a toxic lectin which has been used immunotherapeutically. This is accomplished by binding the alpha-peptide chain of ricin, which is responsible for toxicity, to the antibody molecule to enable site-specific delivery of the toxic effect.

Toxins are poisonous substances produced by plants, animals, or microorganisms that, in sufficient dose, are often lethal. Diphtheria toxin is a protein produced by Corynebacterium diphtheriae. This toxin consists of an alpha and beta subunit which, under proper conditions, can be separated. The toxic component can be bound to antibody and used for site-specific delivery to the primary underlying cause of the inflammatory response.

Examples of radioisotopes which can be bound to specific antibody for therapeutic purposes, used according to the method of the invention, are ¹²⁵I, ¹³¹I, ⁹⁰Y, ⁶⁷Cu, ²¹⁷Bi, ²¹¹At, ²¹²Pb, ⁴⁷Sc, and ¹⁰⁹Pd.

Anti-microbials are substances which inhibit such infectious microorganisms as bacteria, viruses, fungi, and parasites. These anti-microbials can be any of those known to those of ordinary skill in the art.

Other therapeutic agents which can be coupled to specific antibodies used according to the method of the invention are known, or can be easily ascertained, by those of ordinary skill in the art.

Preparations of the imaging immunoglobulins for parenteral administration include sterile aqueous or non-aqueous solutions, suspensions, and emulsions. Examples of non-aqueous solvents are propyleneglycol, polyethyleneglycol, vegetable oil such as olive oil, and injectable organic esters such as ethyloleate. Aqueous carriers include water, alcoholic/aqueous solutions, emulsions or suspensions, including saline and buffered media, parenteral vehicles including sodium chloride solution, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present, such as, for example, antimicrobials, anti-oxidants, chelating agents, and inert gases and the like. See, generally, Remington's Pharmaceutical Science, 16th ed., Mac Eds, 1980.

This invention can be utilized to delineate inflammation at a wide variety of body sites and to diagnose inflammation resulting from a variety of causes such as, but not limited to, infections with parasites, microbes, viruses or fungi; trauma; autoimmune processes; or tumors. The invention is also useful as a means to evaluate the efficacy of, and responses to, therapeutic treatment of inflammation. The diversity of body sites at which inflammation may be identified are exemplified by, but not limited to, muscle, vascular walls, abdomen, groin, and other organ sites.

The above disclosure generally describes the present invention. A more complete understanding can be obtained by reference to the following specific examples.

### COMPARATIVE EXAMPLE 1

### IMAGING OF INFLAMMATION WITH RADIOLABELED PREPARATIONS

Deep thigh inflammation was created in 250 gm Sprague-Dawley rats by the injection of 10⁸ viable E. coli, Staphylococcus aureus, or Pseudomonas aeruginosa suspended in 0.1 ml of phosphate-buffered saline. Twenty-four hours later, at a time when each animal had clearly evident inflammation of the thigh, each animal was injected via the tail vein with 65 ug of a commercially available, intravenous, polyclonal human immunoglobulin preparation (Sandoglobulin®) labeled with 100 µCi of ¹²⁵I (Iodogen®, Pierce Chemical Co., Rockford, Il.) ; 100 µCi of ⁶⁷Ga; and 65 µg of human albumen labeled with 100 µCi of ^{99m}Te. Thus, each animal was injected with three different isotopes, enabling three different approaches to inflammation imaging to the same animal via a gamma counter. Following injections of these various agents, images were obtained at 1, 3, 19, and 24 hours. Figures 1 and 2 illustrate two representative experiments involving three animals in each experiment. Note that the same three animals were imaged at each time point. These images show that there was essentially no imaging of the inflammatory lesion with ⁶⁷Ga (considered the standard inflammatory scanning technique); minimal transient imaging with the ^{99m}Tc labeled albumen; but obvious, increasing visualization of the site of inflammation with the ¹²⁵I-labeled polyclonal human immunoglobulin preparation.

In addition to imaging, radioactive counts and count densities were determined over the inflamed site and the non-inflamed contralateral thigh for each isotope at each of the time points of imaging. These values are expressed in Tables 1 and 2 (which correspond to comparative Figures 1 and 2, respectively). The same results were observed quantitatively: a transient accumulation at the site of inflammation with albumen; minimal if any accumulation with gallium; and a more sustained, increasing accumulation of labeled immunoglobulin.

**Table 1**

| #3/1 hr | | | |
|---|---|---|---|
| inflamed leg | 683/7 | 1791/19 | 2054/23 |
| uninflamed leg | 370/5 | 438/6 | 758/10 |

| #3/3 hr | | | |
|---|---|---|---|
| inflamed leg | 831/11 | 1792/24 | 1195/16 |
| uninflamed leg | 238/4 | 319/5 | 273/5 |

| #3/19 hr | | | |
|---|---|---|---|
| inflamed leg | 677/5 | 4491/35 | 1014/12 |
| uninflamed leg | 307/3 | 638/7 | 341/3 |

| #3/24 hr | | | |
|---|---|---|---|
| inflamed leg | 754/5 | 4148/28 | 897/8 |
| uninflamed leg | 232/3 | 503/6 | 240/3 |
| *Data expressed as counts in area of interest (numerator)/count density in counts/pixel (denominator). | | | |

**Table 2***

| Animal/Time | Gallium-67 | ¹²⁵I-Immunoglobulin | ^{99m}Tc-Albumin |
|---|---|---|---|
| #4/1 hr | | | |
| inflamed leg | 937/12 | 2085/27 | 2051/27 |
| uninflamed leg | 290/6 | 633/12 | 599/13 |

| #4/3 hr | | | |
|---|---|---|---|
| inflamed leg | 868/11 | 3032/41 | 1853/25 |
| uninflamed leg | 369/11 | 696/10 | 434/6 |

| #4/19 hr | | | |
|---|---|---|---|
| inflamed leg | 567/4 | 4576/39 | 1208/13 |
| uninflamed leg | 286/4 | 603/9 | 406/6 |

| #4/24 hr | | | |
|---|---|---|---|
| inflamed leg | 638/5 | 4437/36 | 980/7 |
| uninflamed leg | 249/3 | 1262/13 | 385/4 |

| #5/1 hr | | | |
|---|---|---|---|
| inflamed leg | 800/12 | 1580/24 | 1556/24 |
| uninflamed leg | 621/7 | 935/10 | 891/10 |

| #5/3 hr | | | |
|---|---|---|---|
| inflamed leg | 1025/12 | 2257/28 | 1388/17 |
| uninflamed leg | 282/6 | 575/13 | 357/8 |

| #5/19 hr | | | |
|---|---|---|---|
| inflamed leg | 730/6 | 4268/30 | 1300/11 |
| uninflamed leg | 308/3 | 656/7 | 389/4 |

| #5/24 hr | | | |
|---|---|---|---|
| inflamed leg | 723/6 | 3226/29 | 755/6 |
| uninflamed leg | 345/4 | 668/8 | 280/3 |

| #6/1 hr | | | |
|---|---|---|---|
| inflamed leg | 1315/14 | 2658/29 | 2519/28 |
| uninflamed leg | 226/3 | 369/6 | 385/6 |

| #6/3 hr | | | |
|---|---|---|---|
| inflamed leg | 1245/12 | 3640/36 | 2353/23 |
| uninflamed leg | 301/5 | 507/8 | 371/6 |

| #6/19 hr | | | |
|---|---|---|---|
| inflamed leg | 609/6 | 8161/52 | 1431/17 |
| uninflamed leg | 205/2 | 282/3 | 294/4 |

| #6/24 hr | | | |
|---|---|---|---|
| inflamed leg | 702/5 | 7354/50 | 749/9 |
| uninflamed leg | 287/4 | 455/6 | 215/3 |
| *Data expressed as counts in area of interest (numerator)/count density in counts/pixel (denominator). | | | |

### COMPARATIVE EXAMPLE 2

In other experiments, carried out as in Example 1, ¹¹¹In coupled to the human polyclonal immunoglobulin instead of ¹²⁵I, yielded excellent images that were brighter and appeared more promptly than did the ¹²⁵I-labeled material. This shows that the imaging method is not restricted to a single label.

### COMPARATIVE EXAMPLE 3

A "dose-response" experiment was carried out utilizing ¹²⁵I-labeled polyclonal human immunoglobulin, in which doses of 65 µg, 650 µg, 5.8 mg, and 122 mg were utilized per animal. The procedures of Example 1 were used. In these experiments, the amount of radioactivity was kept constant (100 µCi), and increasing amounts of "cold" immunoglobulin were added to increase the amount of protein injected. Thus, quantities approximately equivalent to a standard dose, 10x standard, 100x standard, and 1000x standard, were injected. All of these concentrations yielded similar images and similar counts over time, suggesting that the receptors for the immunoglobulin were not saturated even at a 1000x dose schedule.Because of the absence of epitopic specificity of the immunoglobulin preparation used, the accumulation of labeled immunoglobulin at the site of inflammation is not due to conventional recognition of epitopic determinants.

### EXAMPLE 1

### INFLAMMATION SITE IMAGING USING NON-SPECIFIC MURINE MONOCLONAL ANTIBODY

Deep thigh inflammation was created in Sprague-Dawley rats as previously described above in comparative Example 1. Twenty-four hours later, at a time when each animal clearly evidenced inflammation in the injected thigh, 65 ug of murine monoclonal antibody labeled with 100 uCi of either ¹²⁵I or ¹¹¹In were injected intravenously via the tail vein. The murine monoclonal antibodies employed in these experiments were of the IgG₁ or IgG₂ subclass and were of three epitopic specificities. The two epitopic specificities which were used were:
1. IgG₁ or IgG₂ antibodies directed against an arsenate hapten not found in mammalian species or bacteria.
2. IgG₁ antibodies specific for lipid A of E. coli.

In these experiments, the anti-arsenate monoclonal antibodies were injected into animals having either E. coli, S. aureus, or P. aeruginosa infection. The anti-E. coli monoclonal antibody was injected into animals bearing staphylococcal or Pseudomonas infection.

All of these labeled immunoglobulin preparations gave similar results. In all animals there appeared an image 1-4 hours post-injection, which peaked in intensity 24-48 hours post-injection, and disappeared completely by 96-120 hours. A representative experiment utilizing the IgG₁ anti-arsenate antibody in an animal with a Pseudomonas infection is shown in Figure 1.

These studies demonstrate that a wide variety of non-specific monoclonal immunoglobulins can be used in this method.

### EXAMPLE 2

### COMPARISON OF NON-SPECIFIC IMMUNOGLOBULIN IMAGING TO SPECIFIC ANTIBODY IMAGING

Pseudomonas aeruginosa Type I deep thigh infection in Sprague-Dawley rats was created as described above. Twenty-four hours later, 0.65 ug of ¹²⁵I-labeled monoclonal antibodies of one of two possible antigenic specificities were injected intravenously via the tail vein. Half of the animals were injected with a murine monoclonal IgG₁ antibody specific for Type I P. aeruginosa. The remaining animals were injected with murine monoclonal IgG₁ antibody specific for arsenate hapten which is an antigen not found in mammalian tissues or bacteria. The anti-arsenate antibody is thus a control preparation with no epitopic specificity for this animal model. Serial images were taken with the gamma camera at 1, 4, 24, 48, 72, 96, 120, 144, and 168 hours after injection of the radiolabeled monoclonal antibodies. In the first twenty-four hours the images obtained with the specific and non-specific antibodies were identical with images appearing as early as 1-4 hours post-injection. However, beginning at 48 hours, the non-specific (anti-arsenate) image began to fade, and had totally disappeared by 72-96 hours. In contrast, the specific (anti-P. aeruginosa) image continued to increase in intensity, peaking at 96-120 hours, and not disappearing until 166 hours had elapsed. Thus, it is possible to differentiate between the non-specific accumulation of immunoglobulin at an inflammation site from the reaction of specific antibody with the underlying cause of the inflammatory response, by following the persistence of the image with time. Specific antibodies remain at the inflammation site longer.

### EXAMPLE 3

A 62-year-old woman with extensive vascular disease underwent a left femoral artery-inferior tibial artery bypass graft on 31/10/86; on 18/11/86 she underwent a right femoral-popliteal arterial bypass graft; and on 2/12/86 a thrombectomy of the right femoral artery. Over the next two weeks, she had a low-grade fever, with minimal findings at her operative site. On 16/12/86, she underwent an immunoglobulin scan which revealed a clear-cut accumulation in the lower portion of her right femoral graft (Figure 2) of radiolabeled immunoglobulin 6 hours post-injection of the reagent. Emergency exploration of this site that evening revealed a mycotic aneurysm of her graft. This was removed, and grew Pseudomonas aeruginosa. In this instance, the scan provided life-saving information non-invasively with no side effects. The results demonstrate the effectiveness of immunoglobulin imaging to diagnose and localize inflammation in the vascular wall.

### EXAMPLE 4

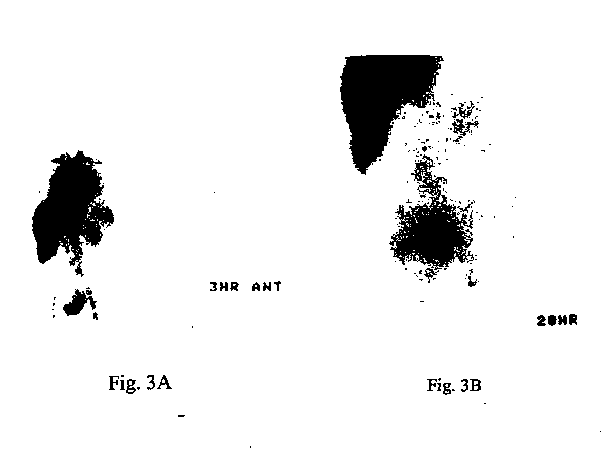

A middle-aged woman presented with fever, lower abdominal pain, and a palpable mass on rectal examination. A computerized tomographic (CT) study of her abdomen revealed no abnormality. An immunoglobulin scan revealed a clear-cut accumulation of radiolabeled immunoglobulin in the lower abdomen as early as 3 hours post-injection (Figure 3) . Surgical exploration revealed a large diverticular abscess at the anatomical site outlined by the immunoglobulin scan.

### EXAMPLE 5

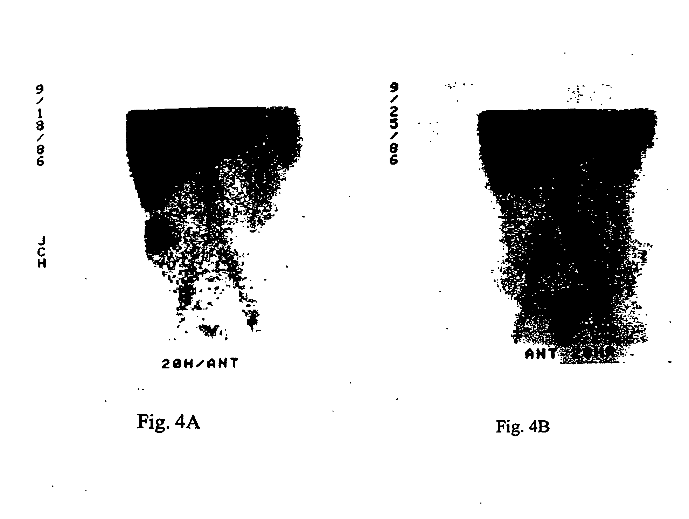

A middle-aged man with longstanding regional enteritis presented with fever and tenderness in his right upper quadrant just below his liver. CT study was non-diagnostic. Initial immunoglobulin scan demonstrated an inflammatory mass just below his liver (Figure 4a) corresponding to the site of pain. He was treated with steroids and broad-spectrum antibiotics with resolution of his symptoms. At the completion of therapy, a repeat immunoglobulin scan revealed no remaining area of inflammation (Figure 4b) ). Therapy was stopped, and the patient remained well. This result demonstrates that immunoglobulin imaging is effective not only as a diagnostic tool but also to determine the efficacy of treatment.

### EXAMPLE 6

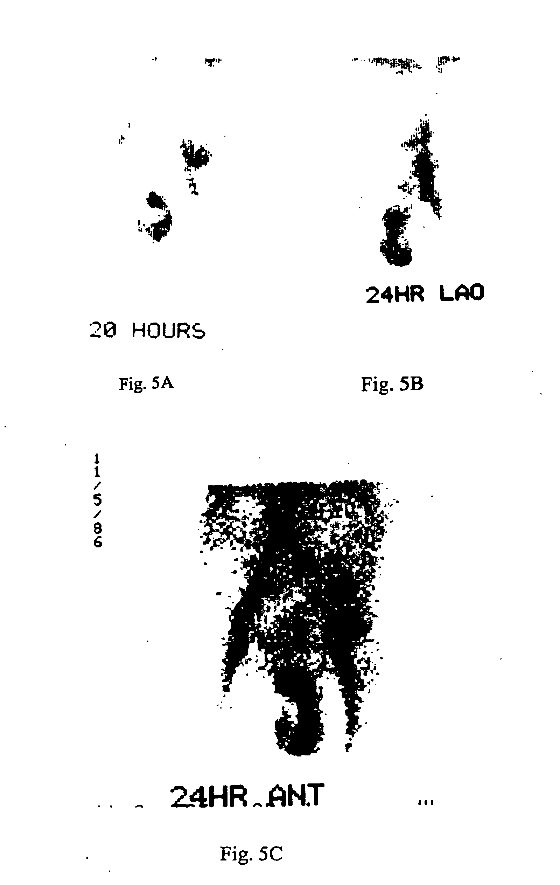

A middle-aged man presented in mid-July 1986 with a left groin mass, fever, and chills of 3-4 days' duration. On 12/7/86, he underwent percutaneous drainage of the groin mass, which yielded purulent material that grew small amounts of mixed bowel flora. He was treated with 7 days of intravenous cefazolin therapy. The initial immunoglobulin scan on 16/7/86 revealed a residual inflammatory mass (Figure 5a) . No further therapy was carried out. The patient continued to drain purulent material from his left groin and to experience discomfort for the next four months. A repeat immunoglobulin scan on 5/11/86 revealed a more extensive inflammatory mass in the same area (Figure 5b). Subsequent surgical exploration revealed an extensive inflammatory mass extending from the sigmoid colon into the groin and up into the retro-peritoneum. Thus, the immunoglobulin imaging technique is also effective in following the course of inflammation over a prolonged period.

## Claims

1. The use of one or more monoclonal antibodies in the preparation of a detectably labelled imaging agent for the diagnosis of inflammation, wherein the antibody or antibodies substantially accumulate at sites of inflammation, but wherein there is no substantial Fab binding to the antigen epitopes at said sites of inflammation.

2. The use of one or more Fc fragments of monoclonal antibodies which accumulate at sites of inflammation in the preparation of a detectably labelled imaging agent for the diagnosis of inflammation, wherein said Fc fragments of monoclonal antibodies have substantially no epitopic specificity for said inflamed sites.

3. A use as claimed in claim 1 or 2 wherein the detectable label is a radioactive isotope or a paramagnetic label.

4. A use as claimed in claim 3, wherein the radioactive isotope is ^{99m}Tc, ¹²³I, ¹³¹I, ¹¹¹In, ⁹⁷Ru, ⁶⁷Cu, ⁶⁷Ga, ⁶⁸Ga, ⁷²As, ⁸⁹Zr or ²⁰¹Tl.

5. A use as claimed in claim 3, wherein the paramagnetic label is ¹⁵⁷Gd, ⁵⁵Mn, ¹⁶²Dy, ⁵²Cr or ⁵⁶Fe.

## Patentansprüche

1. Verwendung eines oder mehrerer monoklonaler Antikörper bei der Herstellung eines nachweisbar, markierten, bildgebenden Mittels zur Diagnose von Entzündungen, wobei der oder die Antikörper sich im wesentlichen am Ort der Entzündung ansammeln, bei dem es aber im wesentlichen keine Fab-Bindung an die Antigenepitope am vorerwähnten Ort der Entzündung gibt.

2. Verwendung eines oder mehrerer Fc-Fragmente monoklonaler Antikörper, die sich an Entzündungsorten ansammeln, bei der Herstellung eines nachweisbar, markierten, bildgebenden Mittels zur Diagnose von Entzündungen, wobei die vorerwähnten Fc-Fragmente von monoklonalen Antikörpern im wesentlichen keine Epitopspezifität für die vorerwähnten entzündeten Orte besitzen.

3. Verwendung nach Anspruch 1 oder 2, **dadurch gekennzeichnet, dass** die nachweisbare Markierung ein radioaktives Isotop oder eine paramagnetische Markierung ist.

4. Verwendung nach Anspruch 3, **dadurch gekennzeichnet, dass** das radioaktive Isotop ^{99m}Tc, ¹²³I, ¹³¹I, ¹¹¹In, ⁹⁷Ru, ⁶⁷Cu, ⁶⁷Ga, ⁶⁸Ga, ⁷²As, ⁸⁹Zr oder ²⁰¹Tl ist.

5. Verwendung nach Anspruch 3, **dadurch gekennzeichnet, dass** die paramagnetische Markierung ¹⁵⁷Gd, ⁵⁵Mn, ¹⁶²Dy, ⁵²Cr oder ⁵⁶Fe ist.

## Revendications

1. Utilisation d'un ou plusieurs anticorps monoclonaux dans la préparation d'un agent d'imagerie marqué de manière détectable pour le diagnostic d'une inflammation, dans laquelle l'anticorps ou les anticorps s'accumule(nt) substantiellement au niveau de sites d'inflammation, mais dans laquelle il n'y a pas de liaison substantielle du Fab aux épitopes de l'antigène au niveau desdits sites d'inflammation.

2. Utilisation d'un ou plusieurs fragments Fc d'anticorps monoclonaux qui s'accumule(nt) au niveau de sites d'inflammation dans la préparation d'un agent d'imagerie marqué de manière détectable pour le diagnostic d'une inflammation, dans laquelle lesdits fragments Fc d'anticorps monoclonaux n'ont substantiellement pas de spécificité d'épitope pour lesdits sites enflammés.

3. Utilisation suivant la revendication 1 ou 2, dans laquelle le marqueur détectable est un isotope radioactif ou un marqueur paramagnétique.

4. Utilisation suivant la revendication 3, dans laquelle l'isotope radioactif est le ^{99m}Tc, le ¹²³I, le ¹³¹I, le ¹¹¹In, le ⁹⁷Ru, le ⁶⁷Cu, le ⁶⁷Ga, le ⁶⁸Ga, le ⁷²As, le ⁸⁹Zr, ou le ²⁰¹Tl.

5. Utilisation suivant la revendication 3, dans laquelle le marqueur paramagnétique est le ¹⁵⁷Gd, le ⁵⁵Mn, le ¹⁶²Dy, le ⁵²Cr ou le ⁵⁶Fe.
